# EUROPÄISCHE PATENTSCHRIFT

(11) **EP 2 101 837 B1**
(45) Veröffentlichungstag und Bekanntmachung des Hinweises auf die Patenterteilung: **15.05.2013**
(21) Anmeldenummer: 07856568.6
(22) Anmeldetag: 11.12.2007
(51) Int. Cl.: A61L 27/54, A61L 29/16, A61L 31/16, A61M 25/00, A61M 31/00

(54) **MATERIALDEPOTKETTE ZUR ABGABE EINES ANTIBAKTERIELLEN WIRKSTOFFMATERIALS**
MATERIAL DEPOT CHAIN FOR RELEASING AN ANTIBACTERIAL ACTIVE AGENT
DÉPÔTCHAÎNE DE MATIÈRES POUR LA LIBÉRATION D'UNE SUBSTANCE ACTIVE ANTIBACTÉRIENNE

(30) Priorität: 20.12.2006 DE 102006060938
(43) Veröffentlichungstag der Anmeldung: 23.09.2009
(73) Patentinhaber: Wolter, Dietmar, 22955 Hoisdorf (DE)
(72) Erfinder: Wolter, Dietmar, 22955 Hoisdorf (DE)
(74) Vertreter: Glawe, Delfs, Moll
(86) Internationale Anmeldenummer: PCT/EP2007/010820
(87) Internationale Veröffentlichungsnummer: WO 2008/077481

(56) Entgegenhaltungen:
- WO-A-00/09177
- DE-A1- 3 115 763
- DE-A1- 3 441 586
- DE-A1- 4 341 442
- FEN G: "[Gentamycin PMMA balls and chains in the treatment of osteomyelitis and soft tissue infections]" FORTSCHRITTE DER MEDIZIN, URBAN UND VOGEL, MUENCHEN, DE, Bd. 98, Nr. 33, 4. September 1980 (1980-09-04), Seiten 1267-1270, XP009103730 ISSN: 0015-8178
- KLEMM K W: "Antibiotic bead chains" CLINICAL ORTHOPAEDICS AND RELATED RESEARCH, PHILADELPHIA, PA; US, Nr. 295, 1. Januar 1993 (1993-01-01), Seiten 63-76, XP009103738 ISSN: 0009-921X

## Beschreibung

Die Erfindung betrifft ein Materialdepotkette zur Abgabe eines antibakteriellen Wirkstoffmaterials in einem menschlichen oder tierischen Körper.

Bei jedem operativen Eingriff kommt es zur Kontamination der Wunde mit Keimen. Trägt der behandelnde Arzt oder die Pflegekraft z.B. keine sterilen Handschuhe und führt nicht in ausreichendem Maße eine Desinfektion durch, so kann es zur Kontamination mit pathologischen Keimen kommen, welche die Wunde besiedeln.

Materialdepots, die das Antibiotikum an die Umgebung, z.B. an das Wundsekret, abgeben und so eine Infektion verhindern oder auch bekämpfen sollen, sind aus offenkundiger Vorbenutzung bekannt. Beispielhaft genannt seien die Gentamycin-Polymethylmethacrylat (PMMA)-Ketten, die auch unter dem Begriff der Septopal^{®}-Ketten dem Fachmann geläufig sind. Die Septopal^{®}-Ketten haben sich bei der Osteomyelitis-Therapie zum therapeutischen Standart entwickelt und werden ebenfalls bei der Infektionsprophylaxe eingesetzt. Die mit dem Antibiotikum Gentamycin versetzten und auf einem Draht fixierten Acryl-Kügelchen sollen durch die anhaltende Abgabe des Antibiotikums vor Ort die Infektion bekämpfen bzw. ihr vorbeugen.

Verglichen mit einer systemischen Antibiotika-Therapie lassen sich mit Septopal^{®}-Ketten am Infektionsort höhere Wirkstoffkonzentrationen erreichen. Es ist bekannt, dass die Abtötung der Keime nicht allein von der Anwesenheit des Antibiotikums in dem das Bakterium umgebende Milieu, sondern auch der lokalen Konzentration des Antibiotikums (Antibiotika-Spiegel) abhängig ist. Die Behandlung mit Septopal^{®}-Ketten ist vielfach noch wirksam, wenn in mikrobiologischen Testungen eine "einfache" Antibiotika-Resistenz nachgewiesen wird, da in den bakteriologischen Testungen zumeist nur solche Antibiotika-Konzentrationen geprüft werden, wie sie im Zuge einer intravenösen Therapie erreichbar sind.

Es ist bekannt, dass dennoch auch bei der Verwendung von antibiotikahaltigen Materialdepots der eingangs genannten Art häufig zu wenig Antibiotikum abgegeben wird um einen hohen Antibiotika-Spiegel zu gewährleisten. Unter ungünstigen Bedingungen, wenn das Antibiotikum vollständig ausgewaschen ist, kann der Antibiotikaträger im Gegenteil sogar zum Bett für Besiedlung mit Bakterien werden.

Dem wird versucht vorzubeugen, indem je nach Stärke des Infektionsgeschehens unterschiedlich viele Kugeln bzw. Septopal^{®}-Ketten oder größere Kugeln implantiert werden, um einen ausreichend hohen Antibiotika-Spiegel zu erhalten. Der Einlage einer Vielzahl an Kugeln bzw. Septopal^{®}-Ketten oder großer Kugeln sind in der Wunde jedoch räumliche Grenzen gesetzt. In jedem Fall ist eine Erweiterung der operativen Maßnahme nötig. Dies ist ein wesentlicher Nachteil, da der Patient dadurch zusätzlich in Mitleidenschaft gezogen wird. Ebenfalls ist die Handhabung bei der Verwendung einer Vielzahl von Kugeln bzw. Septopal^{®}-Ketten umständlich, da jede Kugel bzw. Kette eingelegt und am Ende der Therapie explantiert werden sollte.

Die Aufgabe der Erfindung besteht in der Schaffung einer Materialdepotkette der eingangs genannten Art, die die vorgenannten Nachteile nicht aufweist.

Die Erfindung löst diese Aufgabe durch ein Materialdepotkette mit den Merkmalen des Anspruchs 1. Vorteilhafte Ausführungsformen der Erfindung sind in den Unteransprüchen offenbart.

Gemäß Anspruch 1 ist die Oberfläche des Materialdepots, über die das antibakterielle Wirkstoffmaterial in den menschlichen oder tierischen Körper abgegeben wird, größer als die Einhüllende des Materialdepots.

Die Einhüllende (auch Envelope) bezeichnet eine um einen Körper gespannte geschlossene Fläche. In Querschnittsdarstellung gilt, dass die Einhüllende jede Kurve einer Kurvenschar in einem Punkt berührt. Beispielsweise wird die Einhüllende einer Kugel mit einem Grundradius r und sich in den Innenraum der Kugel erstreckenden Rillen bestimmt durch die Kugeloberfläche, wie sie sich aus dem Grundradius ergibt (4πr²).

Die Erfindung hat erkannt, dass die Menge eines antibakteriellen Wirkstoffmaterials, das über die Oberfläche des Materialdepots abgegeben wird, von dem Gehalt des Wirkstoffmaterials im Träger bzw. der Beladung des Trägermaterials mit dem Wirkstoffmaterial abhängig ist und diesem Gehalt bzw. der Beladung durch das Trägermaterial vorgegebene Obergrenzen gesetzt sind. Die Erfindung hat ebenfalls erkannt, dass das Antibiotikum aus dem Trägermaterial nur aus der obersten Schicht des Trägermaterials herausgelöst wird. Bei Untersuchungen von explantierten Septopal^{®}-Ketten konnte nachgewiesen werden, dass das antibiotische Material Gentamycin lediglich aus dem wenige Millimeter dicken äußeren Bereich der PMMA-Kugeln herausgelöst wird, während die tieferen Schichten gleich bleibende Antibiotika-Anteile aufweisen. Die Erfindung hat des Weiteren erkannt, dass durch eine Vergrößerung der Oberfläche des Materialdepots mehr Antibiotikum pro Zeiteinheit abgegeben werden kann, d.h. ein höherer Antibiotika-Spiegel erreicht werden kann. Einer Antibiotika-Unterdosierung kann dadurch wirksam entgegengewirkt werden. Die hohe lokale Antibiotikakonzentration führt zu effektiver Abtötung nicht nur der empfindlichen, sondern auch partiell resistenter Keime.

Die Erfindung hat ebenfalls erkannt, dass das erfindungsgemäße Materialdepot nicht zu einer möglichen lokalen Antibiotika-Überdosierung im Implantationsbereich führt.

Es hat sich gezeigt, dass das aus dem Materialdepot in die Wundflüssigkeit abgegebene Antibiotikum in der Wundflüssigkeit durch die Dynamik der Wundflüssigkeit, z.B. durch Lagewechsel des Patienten oder durch Muskeltätigkeit, verteilt wird und sich so ein für den Patienten abgesenkter Antibiotika-Spiegel einstellt. Sofern im Einzelfall eine Überdosierung nicht ausgeschlossen werden kann oder eine verzögerte Freisetzung des Antibiotikums erwünscht ist, ist es zweckmäßig die Geschwindigkeit der Antibiotika-Mengenabgabe entsprechend einzustellen. Dies kann durch geeignete Mengenverhältnisse des Antibiotikums im Materialdepot und/oder im Materialdepot eingebaute Zusatzstoffe erfolgen, die die Freisetzung des Antibiotikums verzögern. Die Geschwindigkeit der Freisetzung kann auch dadurch gesteuert werden, indem das Materialdepot unterschiedlich stark gepresst wird oder z.B. schaumförmig aufgebaut wird.

Die Vergrößerung der Oberfläche des Materialdepots kann durch verschiedenste Oberflächenstrukturen, beispielsweise Vertiefungen, muldenförmigen Ausnehmungen, Einstülpungen und Ausstülpungen wie Furchen, Falten, Schlitze, Lamellen, Waben und Bohrungen erfolgen. Die Furchen, Falten und Schlitze verlaufen im bzw. an der Oberfläche des Materialdepots in Längsrichtung.

Vorteilhafterweise beschädigen, reizen oder beeinträchtigen die Oberflächenstrukturen das Gewebe vor Ort nicht. So weist das Materialdepot vorteilhafterweise keine scharfen Kanten auf. Dies ist ebenfalls bei dem Entfernen der nachfolgend noch näher beschriebenen Materialdepotkette von Vorteil, da beim Herausziehen der Kette somit eine Verletzung des Gewebes, der Nerven und Gefäße und ein Nachbluten weitestgehend vermieden werden kann. Es verlaufen die Oberflächenstrukturen, insbesondere die Furchen, Falten und Schlitze längs der vorgesehenen Entnahmerichtung. Bei den Septopal^{®}-Ketten, die in technisch abgewandelter Form gemäß den Ansprüchen verwendet werden können, ist z.B. die Entnahmerichtung durch die Zugrichtung der Kette vorgegeben. Die erfindungsgemäße Anordnung der Furchen, Falten und Schlitze parallel der vorgesehenen Zugrichtung unterstützt das ungehinderte Herausgleiten der Kette beim Herausziehen aus der Wunde.

Die Tiefen oder Höhen der Oberflächenstrukturen sind zweckmäßigerweise so gewählt, dass einerseits die Festigkeit des Trägermaterials nicht beeinträchtigt ist und beispielsweise ein unerwünschter Abbruch von Trägermaterial beim Implantieren oder Explantieren des Materialdepots oder der Repositionierung vermieden wird, andererseits die Materialdicke zwischen den benachbarten Strukturen des Materialdepots so stark ist, dass eine möglichst effektive Wirkstoffabgabe aus dem Trägermaterial in der Wunde erfolgt. So verfügen z.B. Lamellen, d.h. lange Scheiben oder Blätter, besonders wenn sie zu mehreren mit ihren Flächen parallel oder annährend parallel zueinander angeordnet und miteinander verbunden sind, über eine am Materialaufwand gemessen sehr große Oberfläche und Stabilität. Berücksichtigt man, dass das antibiotische Material Gentamycin, aus dem Trägermaterial PMMA lediglich aus einem wenige Millimeter dicken Hüllenbereich nach der Implantation in der Wunde herausgelöst wird, sollte die Materialdicke zwischen den benachbarten Strukturen des Materialdepots vorzugsweise nur wenige Millimeter, vorzugsweise höchstens 5 mm, weiter vorzugsweise 3 mm, besonders bevorzugt 1 bis 3 mm betragen.

Die Strukturierung des erfindungsgemäßen Materialdepots erlaubt es die Oberfläche des Materialdepots gegenüber der Einhüllenden mindestens um den Faktor 1,1, 1,2, 1,3, 1,4, 1,5, 1,6, 1,7, 1,8, 1,9 oder um ein Vielfaches (mindestens um den Faktor 2, 3, 4, 5, 6, 7, 8, 9, 10, 11-100, 100-200, 200-300, 300-400, 400-500, 500-600, 600-700, 700-800, 800-900 oder 900-1000) zu erhöhen.

Zweckmäßigerweise ist das antibakteriell wirkende Material ein Antibiotikum, wobei besonders zweckmäßig Gentamycin ist oder ein desinfizierender Stoff. Alternativ zu oder in Kombination mit Gentamycin können auch weitere Antibiotika, beispielsweise Vanvomycin und/oder Clindamycin verwendet werden.

Das antibakteriell wirkende Material kann in einem resorbierbaren Trägermaterial (z.B. Polyglycolid-Lactid oder Collagen) oder in einem nicht resorbierbaren Material (z.B. PMMA) eingeschlossen sein. Vorzugsweise ist das Materialdepot an seiner Oberfläche, zumindest teilweise, offenporig. Die offenen Poren erlauben eine erleichterte Abgabe des antibakteriellen Wirkstoffmaterials in der Wunde.

Die durch die Einhüllende gebildete geometrische Grundform des Materialdepots ist vorzugsweise eine Form, die ausgewählt ist aus der Gruppe bestehend aus Zylinder, Kugel, Kegel sowie der Kombination dieser Grundformen wie der Tropfenform. Grundsätzlich ist jede Grundform bevorzugt, die es erlaubt das Materialdepot für den Patienten schonend zu implantieren und/oder zu explantieren. "Schonend" bedeutet insbesondere, dass die Wundöffnung zum implantieren und/oder explantieren des Materialdepots möglichst klein gehalten werden kann, gleichzeitig jedoch eine leichte Entfernung trotzdem gewährleistet ist.

Gegenstand vorliegender Erfindung ist eine Materialdepotkette, die mindestens zwei erfindungsgemäße Materialdepots als Kettenglieder aufweist. Die Kettenglieder können auf einem zentralen Draht angeordnet sein. Die Kettenglieder können z.B. eine sphärische Grundform, analog der aus dem Stand der Technik bekannten Septopal^{®}-Ketten, oder eine Walzenform, beispielsweise eine Vollzylinderform, aufweisen. Die benachbarten Kettenglieder können auch zueinander korrespondierende Flächen aufweisen. Die Flächen können z.B. konvex-konkav ausgebildet sein. Dadurch ist auch bei einem geringen Abstand zwischen den benachbarten Kettengliedern eine gelenkartige Bewegung der Kettenglieder möglich, die es erlaubt die Kette leichter zu implantieren, bei Bedarf zu (re-)positionieren oder zu entfernen. Derartige Kettenformen haben den Vorteil, dass sie flexibel sind und sich den lokalen Gegebenheiten der Wunde anpassen lassen.

Die Erfindung wird im folgenden anhand von vorteilhaften Ausführungsformen unter Bezugnahme auf die beigefügten Zeichnungen beschrieben. Es zeigen:
- Fig. 1: ein erfindungsgemäßes kugelförmiges Materialdepot mit Rillen;
- Fig. 2: eine Materialdepotkette mit Kugeln; und
- Fig. 3: eine Materialdepotkette aus mehreren Materialdepots in Walzenform mit einer konvex / konkaven Kontaktflächengestaltung.

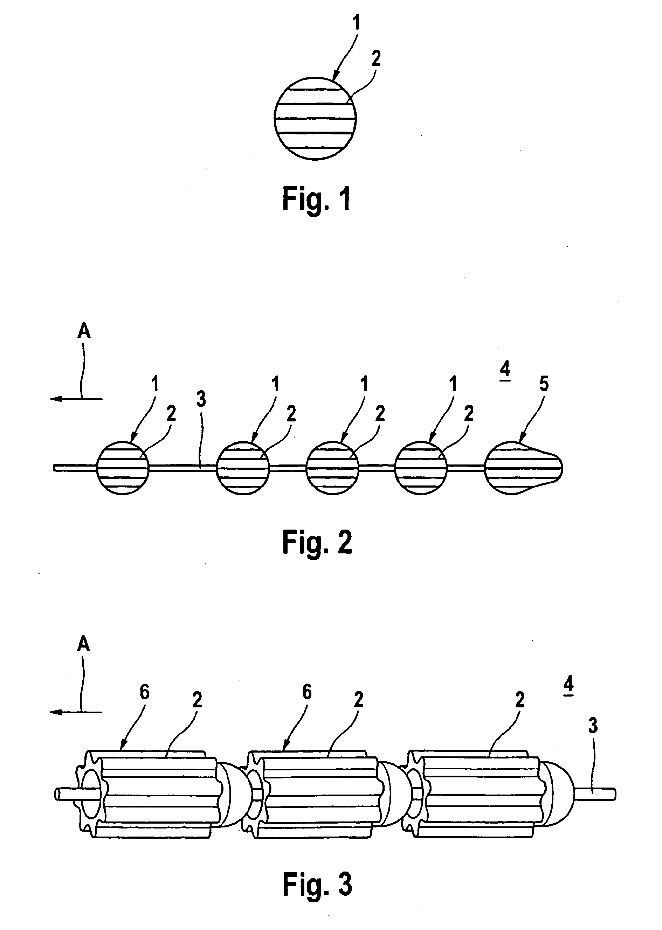

In Figur 1 ist ein kugelförmiges Materialdepot 1 mit parallelen Rillen 2 gezeigt. Das Materialdepot 1 enthält ein antibakterielles Wirkstoffmaterial und gibt dieses über seine offenporige Oberfläche in einem menschlichen oder tierischen Körper ab (nicht dargestellt).

Figur 2 zeigt eine Materialdepotkette 4 mit mehreren Materialdepotkugeln 1 gemäß Figur 1, die auf einem zentralen Draht 3 fixiert sind. Die Materialdepots 1, 5 weisen Rillen 2 auf, die zueinander und in Zugrichtung gemäß Pfeil A parallel angeordnet sind.

Eine weitere Materialdepotkette 4 mit mehreren walzenförmigen Materialdepots 6 die auf einem zentralen Draht 3 fixiert sind, ist in Figur 3 dargestellt. Die walzenförmigen Materialdepots 6 weisen an Stelle der bei einem Zylinder üblichen gegenüberliegenden planen Flächen einander korrespondierende konkav-konvexe Flächen auf. Dadurch ist auch bei einem geringen Abstand zwischen den benachbarten Kettengliedern 6 eine gelenkartige Bewegung der Kettenglieder möglich.

## Patentansprüche

1. Materialdepotkette mit mindestens zwei Materialdepots (1, 5, 6) zur Abgabe eines antibakteriellen Wirkstoffmaterials in einem menschlichen oder tierischen Körper über die Oberfläche des Depots (1, 5, 6), **dadurch gekennzeichnet, dass** die Oberfläche des Materialdepots (1, 5, 6), über die das antibakterielle Wirkstoffmaterial in den menschlichen oder tierischen Körper abgegeben wird, größer ist als die Einhüllende des Materialdepots (1, 5, 6) und dass die Oberfläche des Materialdepots (1, 5, 6) Vertiefungen (2) aufweist, die sich längs der Kette erstrecken.

2. Materialdepotkette (1, 5, 6) nach Anspruch 1, **dadurch gekennzeichnet, dass** die Oberfläche des Materialdepots (1, 5, 6) mindestens um den Faktor 1,1, vorzugsweise mindestens um den Faktor 2, besonders bevorzugt mindestens um den Faktor 3 größer ist als die Einhüllende des Materialdepots (1, 5, 6).

3. Materialdepotkette (1, 5, 6) nach Anspruch 1 oder 2, **dadurch gekennzeichnet, dass** das antibakterielle Wirkstoffmaterial in einem vom Körper zu resorbierenden Trägermaterial und/oder ein vom Körper nicht zu resorbierenden Trägermaterial eingeschlossen ist.

4. Materialdepotkette (1, 5, 6) nach einem der Ansprüche 1 bis 3, **dadurch gekennzeichnet, dass** die Materialdicke zwischen benachbarten Vertiefungen (2) des Materialdepots (1, 5, 6) 5 mm, vorzugsweise 3 mm, besonders bevorzugt 1 bis 3 mm nicht überschreitet.

5. Materialdepotkette (1, 5, 6) nach einem der vorangehenden Ansprüche, **dadurch gekennzeichnet, dass** das Materialdepot (1, 5, 6) an seiner Oberfläche, zumindest teilweise, offenporig ist.

6. Materialdepotkette (1, 5, 6) nach einem der vorangehenden Ansprüche, **dadurch gekennzeichnet, dass** das die geometrische Grundform des Materialdepots (1, 5, 6) ausgewählt ist aus der Gruppe bestehend aus Zylinder, Kugel, Kegel sowie der Kombination dieser Grundformen wie der Tropfenform.

7. Materialdepotkette (4) nach einem der Ansprüche 1 bis 6, **dadurch gekennzeichnet, dass** die gegenüberstehenden Flächen der benachbarten Materialdepotkettenglieder (6) der Form nach, zumindest teilweise, korrespondieren.

8. Materialdepotkette (4) nach Anspruch 7, **dadurch gekennzeichnet, dass** die gegenüberstehenden Flächen der benachbarten Materialdepotkettenglieder (6) konkav-konvex ausgebildet sind.

## Claims

1. A material depot chain comprising at least two material depots (1, 5, 6) for dispensing an antibacterial active ingredient material in a human or animal body via the surface of the depot (1, 5, 6), **characterized in that** the surface of the material depot (1, 5, 6) via which the antibacterial active ingredient material is dispensed into the human or animal body is larger than the envelope of the material depot (1, 5, 6) and that the surface of the material depot (1, 5, 6) comprises depressions (2) that extend in a longitudinal direction of the chain.

2. The material depot chain (1, 5, 6) as claimed in claim 1, **characterized in that** the surface of the material depot (1, 5, 6) is larger than the envelope of the material depot (1, 5, 6) by at least a factor of 1.1, preferably by at least a factor of 2, particularly preferably by at least a factor of 3.

3. The material depot chain (1, 5, 6) as claimed in claim 1 or 2, **characterized in that** the antibacterial active ingredient material is enclosed in a carrier material which is intended to be reabsorbed by the body and/or a carrier material which is not intended to be reabsorbed by the body.

4. The material depot chain (1, 5, 6) as claimed in one of claims 1 to 3, **characterized in that** the material thickness between adjacent depressions (2) of the material depot (1, 5, 6) does not exceed 5 mm, preferably 3 mm, particularly preferably 1 to 3 mm.

5. The material depot chain (1, 5, 6) as claimed in one of the preceding claims, **characterized in that** the material depot (1, 5, 6) has, at least in parts, open pores on its surface.

6. The material depot chain (1, 5, 6) as claimed in one of the preceding claims, **characterized in that** the basic geometric shape of the material depot (1, 5, 6) is selected from the group composed of cylinder, sphere, cone and the combination of these basic shapes such as the teardrop shape.

7. The material depot chain (4) as claimed in one of claims 1 to 6, **characterized in that** the opposing faces of the adjacent material depot chain elements (6) have a corresponding shape, at least in part.

8. The material depot chain (4) as claimed in claim 7, **characterized in that** the opposing faces of the adjacent material depot chain elements (6) have a concave-convex design.

## Revendications

1. Chaîne de dépôts de matériaux avec au moins deux dépôts de matériaux (1, 5, 6) pour la distribution d'un principe actif antibactérien dans un corps humain ou animal sur la surface du dépôt (1, 5, 6), **caractérisée en ce que** la surface du dépôt de matériau (1, 5, 6), par l'intermédiaire de laquelle le principe actif antibactérien est distribué dans le corps humain ou animal, est plus grande que l'enveloppe du dépôt de matériau (1, 5, 6) et **en ce que** la surface du dépôt de matériau (1, 5, 6) comprend des creux (2) qui s'étendent le long de la chaîne.

2. Chaîne de dépôts de matériaux (1, 5, 6) selon la revendication 1, **caractérisée en ce que** la surface du dépôt de matériaux (1, 5, 6) est plus grande, d'au moins un facteur de 1,1, de préférence d'au moins un facteur 2, plus particulièrement d'au moins un facteur 3, que l'enveloppe du dépôt de matériaux (1, 5, 6).

3. Chaîne de dépôts de matériaux (1, 5, 6) selon la revendication 1 ou 2, **caractérisée en ce que** le principe actif antibactérien est incorporé dans un matériau support résorbable par le corps et/ou un matériau support non résorbable par le corps.

4. Chaîne de dépôts de matériaux (1, 5, 6) selon l'une des revendications 1 à 3, **caractérisée en ce que** l'épaisseur du matériau entre des creux (2) adjacents du dépôt de matériau (1, 5, 6) ne dépasse pas 5 mm, de préférence 3 mm, plus particulièrement 1 à 3 mm.

5. Chaîne de dépôts de matériaux (1, 5, 6) selon l'une des revendications précédentes, **caractérisée en ce que** le dépôt de matériau (1, 5, 6) présente des pores ouverts au moins sur une partie de sa surface.

6. Chaîne de dépôts de matériaux (1, 5, 6) selon l'une des revendications précédentes, **caractérisée en ce que** la forme géométrique de base du dépôt de matériau (1, 5, 6) est sélectionnée parmi le groupe constitué d'une forme cylindrique, sphérique, conique ainsi que de la combinaison de ces formes de base comme la forme d'une goutte.

7. Chaîne de dépôts de matériaux (4) selon l'une des revendications 1 à 6, **caractérisée en ce que** les surfaces opposées des maillons de la chaîne de dépôts de matériaux (6) correspondent au moins partiellement en ce qui concerne la forme.

8. Chaîne de dépôts de matériaux (4) selon la revendication 7, **caractérisée en ce que** les surfaces opposées des maillons de la chaîne de dépôts de matériaux (6) présentent une forme concave-convexe.
